# EUROPEAN PATENT APPLICATION

(11) **EP 2 325 637 A1**
(43) Date of publication of application: **25.05.2011**
(21) Application number: 09176799.6
(22) Date of filing: 23.11.2009
(51) Int. Cl.: G01N 33/50

(54) **Novel Rab binding partners of Myosin Va**

(71) Applicant: University College Cork - National University of Ireland, Cork, Cork (IE)
(72) Inventor: McCaffrey, Mary, Cork (IE); Lindsay, Andrew, Cork (IE)
(74) Representative: Gates, Marie Christina Esther

(57) **Abstract**

A polynucleotide sequence encoding a myosin Va Rab binding domain, a pharmaceutical composition comprising the polynucleotide or a peptide encoded by the same for treating or preventing a disease, the use of the composition in the manufacture of medicament to treat or prevent a disease, and a method for screening compounds to identify therapeutic candidates for the treatment of a disease associated with myosin Va malfunction.

## Description

### Field of the Invention

The invention relates to a method for screening compounds to identify therapeutic candidates for the treatment of a disease associated with myosin Va malfunction. The invention also relates to pharmaceutical composition comprising a peptide or nucleic acid encoding a myosin Va Rab-binding domain to treat cells exhibiting abnormal cargo transport to the cell surface.

### Background to the Invention

Rab proteins, as part of the Ras superfamily of small GTPases, are typified by a highly conserved three-dimensional crystal structure in the guanine nucleotide binding domain, consisting of a central six-stranded β-sheet enclosed by 5 α-helices. Rab proteins are present on all compartments of the endomembrane system (endoplasmic reticulum, Golgi, endosomes, lysosomes), the plasma membrane (including cell junctions and focal adhesions), mitochondria and centrioles. The Rab GTPases help regulate a vast array of basic cellular functions from macromolecular homeostasis to growth control. They also control anterograde and retrograde trafficking between compartments to coordinate delivery and membrane recycling and also subcompartmentalise organelles by organising specific membrane domains. In addition, Rab proteins are found upstream and downstream of signalling cascades and can impact gene expression and growth control, for example, Rab5 is implicated in EGF signalling.

Myosin Va is a highly processive actin-based motor protein, and is one of three mammalian myosin V isoforms. It is broadly expressed but is enriched in neuronal cells, testes, and melanocytes. It has been implicated in a wide range of cellular functions including secretory vesicle transport, cell division, organelle inheritance, and cytoplasmic mRNA transport. Recently it has also been proposed to play a role in the nucleus. People who lack functional myosin Va suffer from a rare autosomal recessive disorder called Griscelli's Syndrome Type I (GS1), which is characterised by hypopigmentation and severe neurological defects. The hypopigmentation is caused by the inability of pigment granules to be transported from melanocytes to keratinocytes in the skin of patients. The cause of the neurological deficiencies is less clear but is likely to be due to defective transport of proteins and/or mRNA into dendritic spines. Myosin Va has been demonstrated to interact indirectly, via melanophilin, with Rab27a in melanocytes. This interaction is dependent on the presence of a melanocyte-specific exon, exon F, in the tail of myosin Va.

Recently it has been demonstrated that another myosin V isoform, myosin Vb, can interact directly with Rab8a and Rab11 (Roland et al., Journal Biol. Chem. (2009) Vol. 284(2), pp. 1213-1223). However, myosin Vb has not been implicated in cancer or any neurological diseases to date. It has also recently been demonstrated that upregulation of myosin Va by Snail (a zinc finger transcriptional factor) is involved in cancer cell migration and metastasis (Lan et al., Intl. J. Cancer, June 2009 epub ahead of publication). However, the information presented in the Lan paper does not describe, in any way, how myosin Va function is regulated in the cytoplasm.

Therefore, a greater understanding of how key regulatory molecules such as the Rab GTPases mediate the recruitment and subsequent movement of these unconventional myosins will help to enhance our knowledge of the underlying mechanisms of, amongst others, neurological deficiencies and tumour cell invasiveness. The data provided herein provides information as to how myosin Va function is regulated, *i.e*. via interaction with certain Rab GTPases.

### Summary of the Invention

The present invention provides a method for screening compounds to identify therapeutic candidates for the treatment of a disease associated with myosin Va malfunction by modulating the interaction between myosin Va and Rab proteins, said method comprising:
contacting a compound with a cell expressing a myosin Va Rab binding domain under conditions which permit binding of a myosin Va Rab binding domain to a Rab protein; and
detecting binding between a myosin Va Rab binding domain and a compound;
wherein binding between a myosin Va Rab binding domain and a compound indicates that said compound is a candidate for modulating the interaction between myosin Va and a Rab protein, and regulating the function of myosin Va in the transport of cellular proteins to a cell surface *in vivo.*

The method may comprise a Rab-binding domain of myosin Va encoded by a polynucleotide and/or a polypeptide selected from the group comprising:
(a) a polynucleotide encoded by SEQ ID NO:1;
(b) a polynucleotide encoding the amino acid sequence as defined by SEQ ID NO:2;
(c) a polynucleotide complementary to a polynucleotide of (a) or (b);
(d) a polypeptide encoded by SEQ ID NO: 2;
(e) amino acid residues 1100 to 1853 of SEO ID NO: 2;
(f) amino acid residues 1100 to 1853 of SEO ID NO: 2 having one or several amino acid additions, substitutions, insertions or deletions; and
(g) an amino acid sequence which hybridises under stringent conditions to any one of (d), (e), or (f);
wherein each of (f) to (g) have Rab protein-binding activity.

It may be desirable that the interaction between the Rab protein and myosin Va is dependent on amino acid residues L1633, M1715, Y1719, Q1750, and Q1753 of SEQ ID NO: 2 or a polynucleotide encoding said amino acid residues.

In one aspect of the present invention, the Rab protein is selected from the group comprising Rab3a, Rab6a, Rab6a', Rab11a, Rab11b, and Rab25.

In a further aspect of the present invention, the compound may be selected from the class of compounds comprising (i) small cell-penetrating peptides homologous to the Rab3, Rab11, and Rab6 interacting domains of myosin Va; (ii) small-molecule inhibitors that selectively block the Rab-binding pockets of myosin Va; (iii) siRNA oligonucleotides to modulate the level of myosin Va expression; and (iv) monoclonal antibodies that bind to and inhibit the functioning of myosin Va.

It is desirable that the present invention provides a polynucleotide sequence encoding a myosin Va Rab binding domain selected from the group comprising:
(a) amino acid residues 1100 to 1853 of SEO ID NO: 2;
(b) amino acid residues 1100 to 1853 of SEQ ID NO: 2 having one or several amino acid additions, substitutions, insertions, or deletions; and
(c) an amino acid sequence which hybridises under stringent conditions to any of (a) or (b);
wherein each of (b) and (c) have Rab protein-binding activity.

A polynucleotide sequence encoding a myosin Va Rab binding domain comprising amino acid residues L1633, M1715, Y1719, Q1750, and Q1753 of SEQ ID NO: 2 or a polynucleotide encoding said amino acid residues.

It is a further object of the present invention to provide a pharmaceutical composition comprising a peptide or nucleic acid encoding a myosin Va Rab-binding domain. The composition may be used to treat cells exhibiting abnormal cargo transport to the cell surface.

It is desirable that the Rab-binding domain of myosin Va is a polynucleotide and/or a polypeptide selected from the group comprising:
(a) a polynucleotide encoded by SEQ ID NO:1;
(b) a polynucleotide encoding the amino acid sequence as defined by SEQ ID NO:2;
(c) a polynucleotide complementary to a polynucleotide of (a) or (b);
(d) a polypeptide encoded by SEQ ID NO: 2;
(e) a polynucleotide sequence encoding a myosin Va Rab binding domain selected from the group comprising:
   (1) amino acid residues 1100 to 1853 of SEQ ID NO: 2;
   (2) amino acid residues 1100 to 1853 of SEQ ID NO: 2 having one or several amino acid additions, substitutions, insertions, or deletions; and
   (3) an amino acid sequence which hybridises under stringent conditions to any of (1) or (3);
   wherein each of (2) and (3) have Rab protein binding activity; and
(f) a polynucleotide sequence encoding a myosin Va Rab binding domain comprising amino acid residues L1633, M1715, Y1719, Q1750, and Q1753 of SEQ ID NO: 2 or a polynucleotide encoding said amino acid residues..

The interaction between a Rab protein and myosin Va may be dependent on amino acid residues L1633, M1715, Y1719, Q1750, and Q1753 of SEQ ID NO: 2 or a polynucleotide encoding said amino acid residues.

It is desirable that the Rab protein is selected from the group comprising Rab3a, Rab6a, Rab6a', Rab11 a, Rab11 b, and Rab25.

The composition according to the present invention may further comprise a pharmaceutically effective amount of an agent which modulates the interaction between myosin Va and a Rab protein. It is desirable that said agent may be selected from the group comprising (i) small cell-penetrating peptides homologous to the Rab3, Rab11, and Rab6 interacting domains of myosin Va; (ii) small-molecule inhibitors that selectively block the Rab-binding pockets of myosin Va; (iii) siRNA oligonucleotides to modulate the level of myosin Va expression; and (iv) monoclonal antibodies that bind to and inhibit the functioning of myosin Va.

In a further aspect of the present invention, there is provided a use of an effective amount of a compound identified by the method described above in the manufacture of a medicament to treat or prevent a disease. The disease may be caused by abnormal cargo transport in eukaryotic cells.

The disease may be selected from the group consisting of Griscelli's syndrome type 1, diseases associated with altered long-term potentiation such as memory loss, depression, Alzheimer's disease, Parkinson's disease, neuropathic pain, epilepsy, drug addiction, and highly metastatic lung, breast, colon, and prostate cancer.

The present invention also provides a vector for use in treating or preventing a disease by gene therapy, the vector comprising a polynucleotide and/or polypeptide sequence selected from any of the group comprising:
(a) a polynucleotide encoded by SEQ ID NO:1;
(b) a polynucleotide encoding the amino acid sequence as defined by SEQ ID NO:2;
(c) a polynucleotide complementary to a polynucleotide of (a) or (b);
(d) a polypeptide encoded by SEQ ID NO: 2;
(e) amino acid residues 1100 to 1853 of SEQ ID NO: 2;
(f) amino acid residues 1100 to 1853 of SEQ ID NO: 2 having one or several amino acid additions, substitutions, insertions or deletions; and
(g) an amino acid sequence which hybridises under stringent conditions to any one of (d), (e), or (f);
wherein each of (f) to (g) have Rab protein-binding activity; and optionally (h) amino acid residues 1100 to 1853 of SEQ ID NO: 2;
(i) amino acid residues 1100 to 1853 of SEQ ID NO: 2 having one or several amino acid additions, substitutions, insertions, or deletions; and
(j) an amino acid sequence which hybridises under stringent conditions to any of (h) or (i);
wherein each of (i) and (j) have Rab protein-binding activity; and optionally a polynucleotide sequence encoding a myosin Va Rab binding domain comprising amino acid residues L1633, M1715, Y1719, Q1750, and Q1753 of SEQ ID NO: 2 or a polynucleotide encoding said amino acid residues.

The composition may be used to treat a disease caused by abnormal cargo transport in eukaryotic cells. It is desirable that said disease may be selected from the group consisting of Griscelli's syndrome type 1, diseases associated with altered long-term potentiation such as memory loss, depression, Alzheimer's disease, Parkinson's disease, neuropathic pain, epilepsy, drug addiction, and highly metastatic lung, breast, colon, and prostate cancer.

Where suitable, it will be appreciated that all optional and/or preferred features of one embodiment of the invention may be combined with optional and/or preferred features of another/other embodiment(s) of the invention.

The work presented herein identifies novel interactions of myosin Va with Rab11, Rab3, and Rab6. These Rab proteins are key regulatory molecules that the applicants believe mediate key spatio-temporal regulation over myosin Va. The Applicants have identified the exact amino acids on myosin Va that mediate the interaction with Rab3 and Rab11. The Applicants have also identified a binding pocket of approximately 50 amino acids that binds to Rab6. The work presented here is the first time that the interactions between myosin Va and Rab3 and Rab6 have been reported. The amino acids mediating the Rab3 and Rab11 interactions have not been identified previously, nor has the Rab6 binding pocket.

### Brief Description of the Drawings

Additional features and advantages of the present invention are described in, and will be apparent from, the detailed description of the invention and from the drawings in which:

**Figure 1(a) to (d)**: Myosin Va interacts with the GTP-bound forms of Rab3, Rab6, and Rab11;

**Figure 2(a)**: The Rab Interacting Domains of Myosin Va;

**Figure 2(b) and Figure 2(c)**: Space filling 3D models of the globular tail domains of Myo2p and human MyoVa, respectively. Locations of residues predicted to mediate the Rab interactions indicated by the annotated lines. (Adapted from Lipatova Z, Tokarev AA, Jin Y, Mulholland J, Weisman L, Segev N. Mol Biol Cell. 2008 Jul 23);

**Figure 2(d)**: *S. cerevisiae* L40 were co-transformed with GAL4-AD fusions of the indicated MyoVa mutants and GAL4-DBD fusions of the Rab GTPases;

**Figure 3**: Colocalisation of mCherry-MyoVa₍₁₁₀₀₋₁₈₅₃₎ with GFP-Rab GTPases;

**Figure 4**: GFP-MyoVa₍₁₁₀₀₋₁₈₅₃₎ colocalises with endogenous Rab6 and Rab11;

**Figure 5**: FRET between mCherry-MyoVa₍₁₁₀₀₋₁₈₅₃₎ and GFP-Rab3, GFP-Rab6 and GFP-Rab11;

**Figure 6(a) and 6(b)**: Knockdown of myosin Va using siMyoVa1 perturbs Rab6 and EEA1 localisation;

**Figure 7**: Knockdown of myosin Va using siMyoVa1 inhibits VSVG transport from the Golgi to the plasma membrane;

**Figure 8(a) and 8(b)**: (a) Knockdown of myosin Va in breast cancer cell lines using siMyoVa1 and (b) reduced migration of MDA-MB-231 cells transfected with siMyoVa 1.

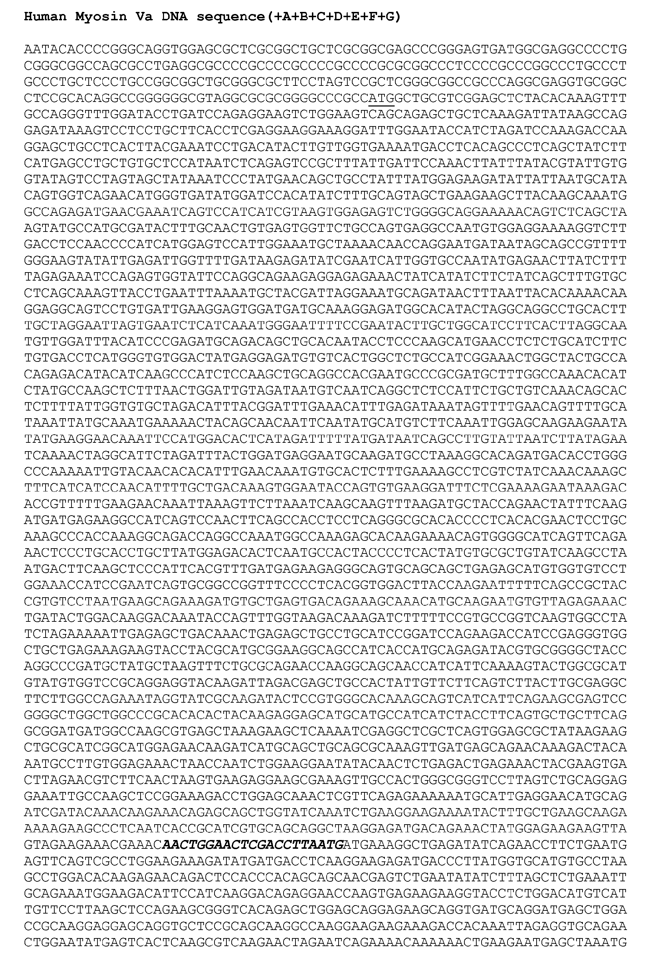

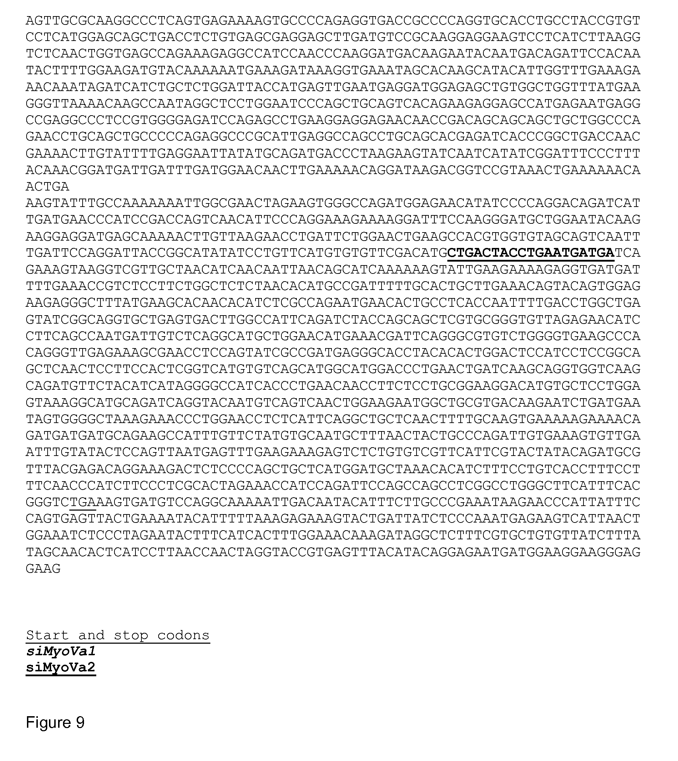

**Figure 9**: Polynucleotide sequence of Myosin Va (SEQ ID NO: 1) with the siRNA target sequences highlighted, and including all 7 exons (A+B+C+D+E+F+G).

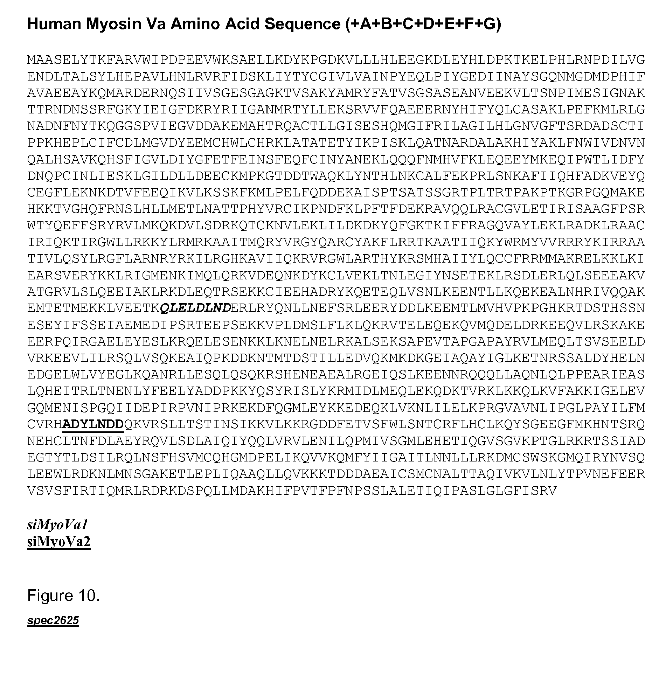

**Figure 10**: Amino acid sequence of Myosin Va (SEQ ID NO: 2) including all 7 exons (A+B+C+D+E+F+G).

### Detailed Description of the Drawings

### Definitions

"Synthetic oligonucleotide" refers to molecules of nucleic acid polymers of 2 or more nucleotide bases that are not derived directly from genomic DNA or live organisms. The term synthetic oligonucleotide is intended to encompass DNA, RNA, and DNA/RNA hybrid molecules that have been manufactured chemically, or synthesized enzymatically *in vitro.*

An "oligonucleotide" is a nucleotide polymer having two or more nucleotide subunits covalently joined together. Oligonucleotides are generally about 10 to about 100 nucleotides. The sugar groups of the nucleotide subunits may be ribose, deoxyribose, or modified derivatives thereof such as OMe. The nucleotide subunits may be joined by linkages such as phosphodiester linkages, modified linkages or by non-nucleotide moieties that do not prevent hybridization of the oligonucleotide to its complementary target nucleotide sequence. Modified linkages include those in which a standard phosphodiester linkage is replaced with a different linkage, such as a phosphorothioate linkage, a methylphosphonate linkage, or a neutral peptide linkage. Nitrogenous base analogs also may be components of oligonucleotides in accordance with the invention. A "target nucleic acid" is a nucleic acid comprising a target nucleic acid sequence. A "target nucleic acid sequence," "target nucleotide sequence" or "target sequence" is a specific deoxyribonucleotide or ribonucleotide sequence that can be hybridized to a complementary oligonucleotide.

An "oligonucleotide probe" is an oligonucleotide having a nucleotide sequence sufficiently complementary to its target nucleic acid sequence to be able to form a detectable hybrid probe:target duplex under high stringency hybridization conditions. An oligonucleotide probe is an isolated chemical species and may include additional nucleotides outside of the targeted region as long as such nucleotides do not prevent hybridization under high stringency hybridization conditions. Non-complementary sequences, such as promoter sequences, restriction endonuclease recognition sites, or sequences that confer a desired secondary or tertiary structure such as a catalytic active site can be used to facilitate detection using the invented probes. An oligonucleotide probe optionally may be labelled with a detectable moiety such as a radioisotope, a fluorescent moiety, a chemiluminescent, a nanoparticle moiety, an enzyme or a ligand, which can be used to detect or confirm probe hybridization to its target sequence. Oligonucleotide probes are preferred to be in the size range of from about 10 to about 100 nucleotides in length, although it is possible for probes to be as much as and above about 500 nucleotides in length, or below 10 nucleotides in length.

A "hybrid" or a "duplex" is a complex formed between two single-stranded nucleic acid sequences by Watson-Crick base pairings or non-canonical base pairings between the complementary bases. "Hybridization" is the process by which two complementary strands of nucleic acid combine to form a double-stranded structure ("hybrid" or "duplex").

"Complementarity" is a property conferred by the base sequence of a single strand of DNA or RNA which may form a hybrid or double-stranded DNA:DNA, RNA:RNA or DNA:RNA through hydrogen bonding between Watson-Crick base pairs on the respective strands. Adenine (A) ordinarily complements thymine (T) or uracil (U), while guanine (G) ordinarily complements cytosine (C).

The term "stringency" is used to describe the temperature, ionic strength and solvent composition existing during hybridization and the subsequent processing steps. Those skilled in the art will recognize that "stringency" conditions may be altered by varying those parameters either individually or together. Under high stringency conditions only highly complementary nucleic acid hybrids will form; hybrids without a sufficient degree of complementarity will not form. Accordingly, the stringency of the assay conditions determines the amount of complementarity needed between two nucleic acid strands forming a hybrid. Stringency conditions are chosen to maximize the difference in stability between the hybrid formed with the target and the non-target nucleic acid.

With "high stringency" conditions, nucleic acid base pairing will occur only between nucleic acid fragments that have a high frequency of complementary base sequences (for example, hybridization under "high stringency" conditions, may occur between homologs with about 85-100% identity, preferably about 70-100% identity). With medium stringency conditions, nucleic acid base pairing will occur between nucleic acids with an intermediate frequency of complementary base sequences (for example, hybridization under "medium stringency" conditions may occur between homologs with about 50-70% identity). Thus, conditions of "weak" or "low" stringency are often required with nucleic acids that are derived from organisms that are genetically diverse, as the frequency of complementary sequences is usually less.

"High stringency" conditions are those equivalent to binding or hybridization at 42°C in a solution consisting of 5xSSPE (43.8g/1NaCl, 6.9 g/1NaH₂PO₄H₂O and 1.85 g/l EDTA, pH adjusted to 7.4 with NaOH), 0.5% SDS, 5xDenhardt's reagent and 100 µg/m1 denatured salmon sperm DNA followed by washing in a solution comprising 0.1xSSPE, 1.0%SDS at 42°C when a probe of about 500 nucleotides in length is used.

"Medium stringency" conditions are those equivalent to binding or hybridization at 42°C in a solution consisting of 5XSSPE (43.8 g/l NaCl, 6.9 g/l NaH₂PO₄H₂O and 1.85 g/l EDTA, pH adjusted to 7.4 with NaOH), 0.5% SDS, 5xDenhardt's reagent and 100 □g/ml denatured salmon sperm DNA followed by washing in a solution comprising 1.0xSSPE, 1.0% SDS at 42°C, when a probe of about 500 nucleotides in length is used.

"Low stringency" conditions are those equivalent to binding or hybridization at 42°C in a solution consisting of 5xSSPE (43.8 g/l NaCl, 6.9 g/l NaH₂PO₄H₂O and 1.85 g/l EDTA, pH adjusted to 7.4 with NaOH), 0.1% SDS, 5xDenhardt's reagent [50xDenhardt's contains per 500ml: 5g Ficoll (Type 400, Pharamcia), 5 g BSA (Fraction V; Sigma)] and 100 □g/ml denatured salmon sperm DNA followed by washing in a solution comprising 5xSSPE, 0.1% SDS at 42°C, when a probe of about 500 nucleotides in length is used.

In the context of nucleic acid *in-vitro* amplification based technologies, "stringency" is achieved by applying temperature conditions and ionic buffer conditions that are particular to that *in-vitro* amplification technology. For example, in the context of PCR and real-time PCR, "stringency" is achieved by applying specific temperatures and ionic buffer strength for hybridisation of the oligonucleotide primers and, with regards to real-time PCR hybridisation of the probe/s, to the target nucleic acid for *in-vitro* amplification of the target nucleic acid.

By "sufficiently complementary" or "substantially complementary" is meant nucleic acids having a sufficient amount of contiguous complementary nucleotides to form, under high stringency hybridization conditions, a hybrid that is stable for detection. Substantially complementary to can also refer to sequences with at least 90% identity to, *e.g*., 95, 96, 97, 98, 99, or 100% identity to, a given reference sequence.

The terms "identical" or percent "identity," in the context of two or more nucleic acids or polypeptide sequences, refer to two or more sequences or subsequences that are the same or have a specified percentage of amino acid residues or nucleotides that are the same (*i.e*., about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection (*see, e.g.,* NCBI web site at ncbi.nlm.nih.gov/BLAST/or the like). Such sequences are then said to be "substantially identical." This definition also refers to, or may be applied to, the compliment of a test sequence. The definition also includes sequences that have deletions and/or additions, as well as those that have substitutions. As described below, the preferred algorithms can account for gaps and the like. Preferably, identity exists over a region that is at least about 25 amino acids or nucleotides in length, or more preferably over a region that is 50-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window" includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see, e.g.,* Current Protocols in Molecular Biology (Ausubel et al., eds. 1987-2005, Wiley Interscience)).

A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. BLAST and BLAST 2.0 are used, with the parameters described herein, to determine percent sequence identity for the nucleic acids and proteins of the invention. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*)*.* These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff & Henikoff, Proc. Natl. Acad. Sci. USA 89:10915 (1989)) alignments (B) of 50, expectation (E) of 10, M=5, N=-4, and a comparison of both strands.

"Nucleic acid" refers to deoxyribonucleotides or ribonucleotides and polymers thereof in either single- or double-stranded form, and complements thereof. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

"RNA and DNA equivalents" refer to RNA and DNA molecules having the same complementary base pair hybridization properties. RNA and DNA equivalents have different sugar groups (i.e., ribose versus deoxyribose), and may differ by the presence of uracil in RNA and thymine in DNA. The difference between RNA and DNA equivalents do not contribute to differences in substantially corresponding nucleic acid sequences because the equivalents have the same degree of complementarity to a particular sequence.

By "preferentially hybridize" is meant that under high stringency hybridization conditions oligonucleotide probes can hybridize their target nucleic acids to form stable probe:target hybrids (thereby indicating the presence of the target nucleic acids) without forming stable probe:non-target hybrids (that would indicate the presence of non-target nucleic acids from other organisms). Thus, the probe hybridizes to target nucleic acid to a sufficiently greater extent than to non-target nucleic acid to enable one skilled in the art to accurately detect the presence of and distinguish these species from other organisms. Preferential hybridization can be measured using techniques known in the art.

The term "short interfering RNA" (siRNA) refer to 21 to 23 nucleotide duplexes which target specific genes. The term used to describe the use of siRNA in silencing gene expression is RNA interference (RNAi). Generally, siRNA duplexes are incorporated into an siRNA-ribonucleo-protein complex (siRNP), which rearranges to the RNA-induced silencing complex (RISC). The RISC targets the mRNA of interest, cleaves the RNA and releases the cleavage products. RISC may be reactivated for a further round of RNA cleavage.

The term "Rab protein binding activity", as used herein, refers to a peptide, a nucleic acid encoding a peptide, a protein, a nucleic acid encoding a protein, and the like, which can bind to or interact with a Rab protein. By this it is meant that a peptide, a protein, and the like, has an affinity for and will bind to a Rab protein.

It should be readily apparent to one of ordinary skill in the art that the examples disclosed herein below represent generalised examples only, and that other arrangements and methods capable of reproducing the invention are possible and are embraced by the present invention.

**Examples**

### Materials and Methods

### Cell Culture

HeLa cells, MDA-MB-231 cells, and MCF7 cells were obtained from the European Collection of Cell Cultures. Cells were maintained in Dulbecco's modified Eagles medium (DMEM; Sigma) supplemented with 10% foetal bovine serum, 100 units/ml penicillin, 100 µg/ml streptomycin, 25mM Hepes, pH7.4, and 2mM glutamine at 5% CO₂.

### Plasmids

Full-length myosin Va was amplified from a Marathon Human Colon cDNA library (Clontech) using Pfu Turbo DNA polymerase (Stratagene) and hMyoVa Fwd (5'-AAAAGAATTCAGCTGCGTCGGAGCTCTACA-3') and hMyoVa Rev (5'-AAAAGTCGACCCTCCCTTCCTTCCATCATT-3') primers. The resulting PCR product was digested with EcoRI and Sall and ligated into the EcoRI/SalI sites of pEGFP-C1 (Clontech). Myosin Va₍₁₁₀₀₋₁₈₅₃₎ was amplified using hMyoVa₍₁₁₀₀₎ Fwd (5'-AAAAGTCGACCCTCCCTTCCTTCCATCATT-3') and hMyoVa Rev. The PCR product was digested with EcoRI and SalI and ligated into the EcoRI/Sall sites of pEGFP-C1, pmCherry-C1, and pGADGH (Clontech). The Rab GTPase yeast two-hybrid and GFP constructs have been described elsewhere.

Site-directed mutagenesis was performed with the Quikchange kit from Stratagene using pEGFP-C1 MyoVa₍₁₁₀₀₋₁₈₅₃₎ as template and the following sense and antisense primers.

### Yeast Two-Hybrid Interactions

**Table 1: Primer name and Primer Sequence used for the Yeast Two-Hybrid interactions**

| **Primer Name** | **Primer Sequence (5' to 3')** |
|---|---|
| M1715S | 5'-gctcctggagtaaaggcagccagatcaggtacaatgtc-3' |
| M1715S_antisense | 5'-gacattgtacctgatctggctgcctttactccaggagc-3' |
| Y1719E | 5'-taaaggcatgcagatcagggagaatgtcagtcaactggaag-3' |
| Y1719E antisense | 5'-cttccagttgactgacattctccctgatctgcatgccttta-3' |
| Q1753R | 5'-ctcattcaggctgctcaacttttgagagtgaaaaagaaaacagatgatga-3' |
| Q1753R_antisense | 5'-tcatcatctgttttctttttcactctcaaaagttgagcagcctgaatgag-3' |
| Q1750A | 5'-ggaacctctcattcaggctgctgcacttttgcaagtgaaaa-3' |
| Q1750A_antisense | 5'-ttttcacttgcaaaagtgcagcagcctgaatgagaggttcc-3' |
| Q1634S | 5'-gcatgctggaacatgaaacgatttcgggcgtgtctgg-3' |
| Q1634S_antisense | 5'-ccagacacgcccgaaatcgtttcatgttccagcatgc-3' |

Briefly, the yeast reporter strain L40 (MATa *trp1 leu2 his3::lexA-His3 URA3::lexA-lacZ)* was co-transformed with the indicated plasmid constructs. Three days post-transformation individual colonies were picked, and an interaction was indicated by the ability of the transformants to grow on minimal (Drop out) medium lacking histidine.

### Antibodies

Antibodies used were anti-MyoVa (Sigma), anti-α tubulin (Sigma), anti-EEA1 (BD Biosciences), anti-GM130 (BD Biosciences), anti-Rab11 (Zymed), anti-Transferrin Receptor (Zymed), anti-Rab6 (Santa Cruz), and anti-β-actin (Sigma).

### Western Blotting

SDS-PAGE (SDS-polyacrylamide gel electrophoresis) was carried out routinely, and cellular proteins separated on the gel were transferred onto nitrocellulose membrane (Whatman). After blocking with 5% fat-free dried milk in Tris-buffered saline (TBS, 50 mM Tris-HCl pH 7.5, 150 mM NaCl), the membrane was incubated for 1 hr at room temperature with primary antibody specific to myosin Va or β-actin at a dilution of 1:1000 or 1:20000, respectively. Then the membrane was washed three times with TBS/0.05% Tween-20 and reacted with IRDye⁸⁰⁰ goat anti-mouse or IRDye⁸⁰⁰ goat anti-rabbit at 1:10000 dilution (Rockland Immunochemicals Inc.) for 1 hour at room temperature. The membrane was washed three times with TBS/0.05% Tween-20 and a final wash with TBS. Signals were visualized using a LiCor Odyssey Infrared Imaging System.

### siRNA transfections

HeLa cells were seeded on 10mm glass coverslips at approximately 20,000 cells/well of a 24-well plate in complete DMEM in order for transfections to be performed the next day. 1µl Oligofectamine (Invitrogen) was diluted in 6.5µl DMEM per transfection and incubated at room temperature (RT) for 5 minutes. Meanwhile 50pmol of the indicated siRNA was made up to 42.5µl with DMEM per transfection. The Oligofectamine/DMEM dilution was added to the siRNA/DMEM dilution, vortexed, and incubated at RT for 15 minutes. The complete media from the HeLa cells was removed, washed once with PBS, and 200µl DMEM plus 2mM L-glutamine and 25mM Hepes, pH7.4 was added. The Oligofectamine/siRNA complex was added to the cells. 4 hours later 1 25µl DMEM supplemented with 30% foetal bovine serum plus 25mM Hepes, pH7.4 and 2mM L-glutamine was added to each well and the cells were incubated for a further 68 hours at 37°C with 5% CO₂. For Western blotting transfections were performed in 6 well plates. 90,000 HeLa cells were seeded per well and transfected with 200pmol of siRNA using 2.5µl Oligofectamine.

Transfection of MDA-MB-231 and MCF7 breast cancer cell lines were performed using the same protocol as above.

**Table 2: siRNA sequences**

| **Name** | **Sequence** |
|---|---|
| siCONTROL | 5'- AACUGGAACUCGACCUUAAUG -3' |
| siMyoVal1 | 5'- AAAACAAGGACUUGCGUCUUC -3' |
| siMyoVa2 | 5'- CUGACUACCUGAAUGAUGA -3' |

### Virus infection and vesicular stomatitis virus G-protein (VSV-G) Transport Assays

For the Venus-VSVG transport assays HeLa cells in 24-well plates were transfected with siRNA, as above, with the exception that after 54 hours the cells were further transfected with 0.1 µg pVenus-VSVG (Addgene) using 2.5µl Effectene (Qiagen) according to the manufacturers instructions. The cells were then incubated for the final 18 hours at 40°C. Subsequently, the media was replaced with complete media plus 50µg/ml cycloheximide (Sigma) and the cells were either fixed or incubated for 30, 60, or 180 minutes at 32°C prior to fixation and labelling.

### Immunofluorescence and confocal microscopy

HeLa cells were fixed with 3% paraformaldehyde (PFA) for 15 minutes at room temperature and quenched with 50mM NH₄Cl. After washing, the cells were permeablised with 0.1% TX-100 and labelled for 1 hour in a humid chamber with the indicated primary antibodies. The coverslips were washed and incubated for a further hour with secondary antibodies, washed again and mounted on microscope slides with Mowiol. The secondary antibodies used were Alexa Fluor⁵⁹⁴-goat anti-rabbit or Alexa Fluor⁵⁹⁴-goat anti-mouse from Molecular Probes. Cells co-transfected with a GFP-fusion and an mCherry-fusion plasmid were fixed with 3% PFA for 15 minutes at RT and mounted on slides with Mowiol.

Fluorescence images were acquired on a Zeiss LSM 510 META confocal microscope and processed with Image Examiner software (Zeiss). Figures were created in Adobe Illustrator version 10. FRET was determined using the sensitised emission method and was calculated using the FRET Analyzer plugin in ImageJ.

### Wound Assay

MDA-MB-231 cells transfected with siCONTROL or siMyoVa1 or siMyoVa2 for 72 hours were grown to approximately 90% confluence. Media was removed and wounded with a sterile 200µl pipette tip drawn through a portion of the cell monolayer to inflict a wound in the sheet of confluent cells. The cells are rinsed extensively with PBS and fresh complete media is then added. Cells were monitored at 0, 12, 24 hours and 48 hours to assess the extent of cell migration following wounding.

### Results

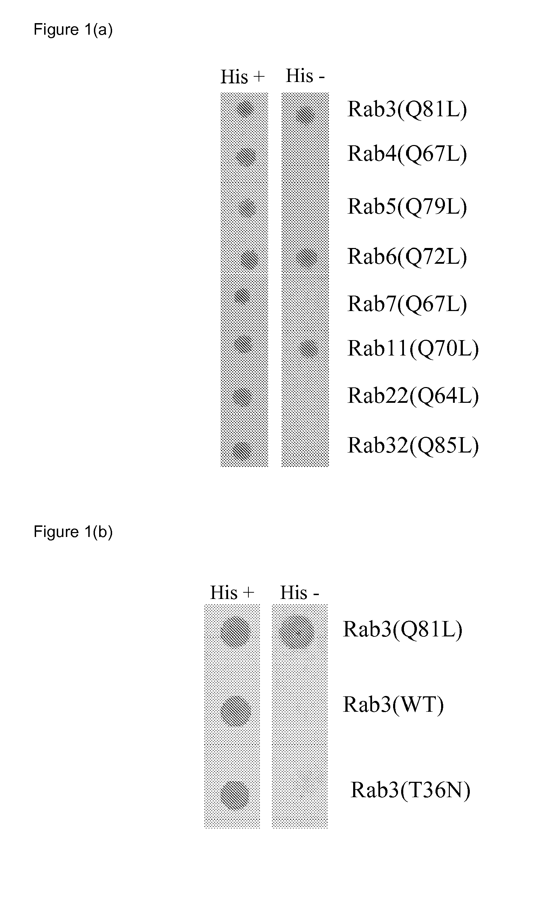

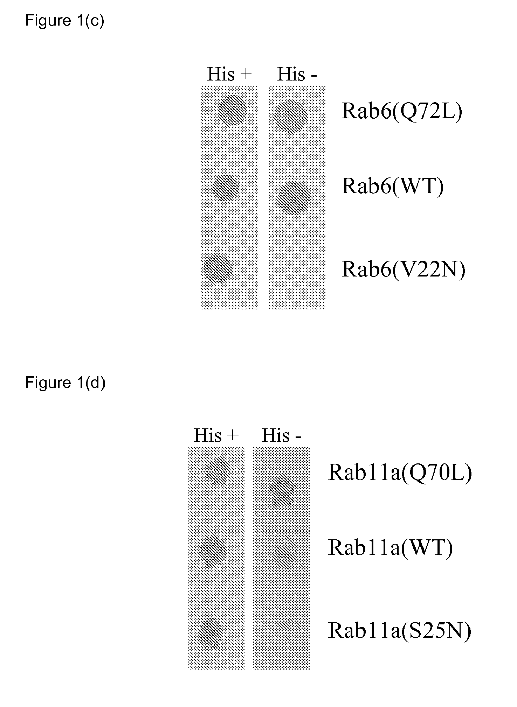

Now referring to **Figure 1**, using the yeast-two hybrid system, it was investigated whether MyoVa₍₁₁₀₀₋₁₈₅₃₎ interacts with the following Rab GTPases listed below. *Saccharomyces cerevisiae (S. cerevisiae)* L40 were co-transformed with a GAL4-AD fusion of MyoVa₍₁₁₀₀₋₁₈₅₃₎ and GAL4-DBD fusions of the following constitutively active Rab GTPase mutants: Rab3(Q81 L), Rab4(Q67L), Rab5(Q79L), Rab6(Q72L), Rab7(Q67L), Rab11 (Q70L), Rab22(Q64L), Rab32(Q85L), or with Rab3(WT), Rab6(WT), Rab11 a(WT), and the following constitutively inactive Rab mutants: Rab3(T36N), Rab6(V22N), and Rab11a(S25N). An interaction between the GAL4-AD fusion of MyoVa₍₁₁₀₀₋₁₈₅₃₎ and GAL4-DBD fusion of a Rab GTPase is indicated by ability of the transformants to grow on media lacking histidine. As illustrated in Figure 1, myosin Va interacts with the constitutively-active forms of Rab3, Rab6, Rab11, (Rab3(Q81L), Rab6(Q72L), Rab11 (Q70L)) and wild-type Rab6 and Rab11a. Myosin Va did not interact with Rab4(Q67L), Rab5(Q79L), Rab7(Q67L), Rab22(Q64L), Rab32(Q85L), Rab3(WT), Rab3(T36N), Rab6(V22N), and Rab11a(S25N) (results not shown).

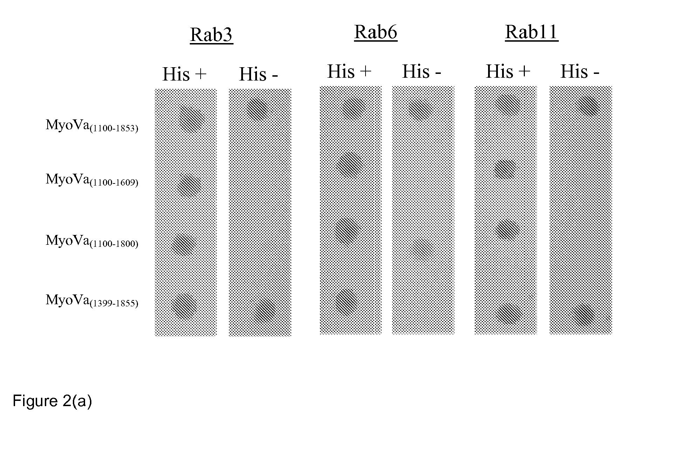

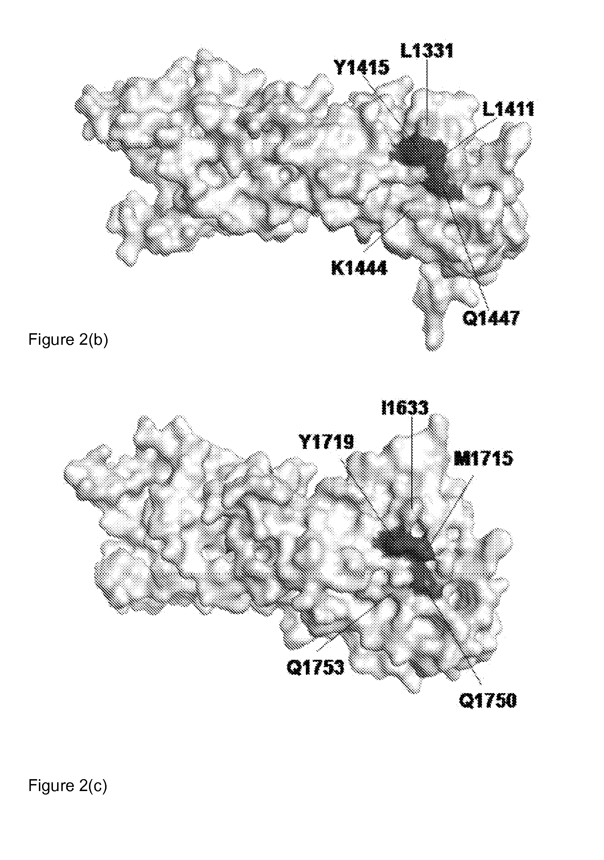

To determine which domains of myosin Va interact with Rab3, Rab6, and Rab11, the yeast two-hybrid system was employed again. *S*. *cerevisiae* L40 were co-transformed with GAL4-AD fusions of MyoVa and GAL4-DBD fusions of the constitutively active mutants of Rab GTPases Rab3, Rab6, and Rab11. As shown in **Figure 2(a)**, the domains of MyoVa₍₁₁₀₀₋₁₈₅₃₎ and MyoVa₍₁₃₉₉₋₁₈₅₅₎ interact with Rab3 and Rab11, while the domains of MyoVa₍₁₁₀₀₋₁₈₅₃₎ and MyoVa₍₁₁₀₀₋₁₈₀₀₎ interact with Rab6. To further define which residues mediate the interaction between myosin Va and the Rab GTPases Rab3, Rab6, and Rab11, several myosin Va mutants were generated in the regions shown above to interact with the Rab proteins. **Figure 2(b)** and **Figure 2(c)** are space filling 3D models of the globular tail domains of Myo2p and human MyoVa, respectively. From work previously carried out by Lipatova *et al.* on the interaction of Ypt31/32 in their GTP-bound form with the GTD domain of Myo2 in yeast, the location of residues in human MyoVa predicted to mediate the Rab interactions were determined as Q1634, M1715, Y1719, Q1750, and Q1753. The one letter amino acid code and position in the amino acid sequence of myosin Va are used here. Each of the predicted residues was mutated to Q1634S, M1715S, Y1719E, Q1750A, and Q1753R. The one-letter nomenclature for amino acids Q, M, Y, S, E, A, and R are short for glutamine, methionine, tyrosine, serine, glutamic acid, alanine, and arginine, respectively.

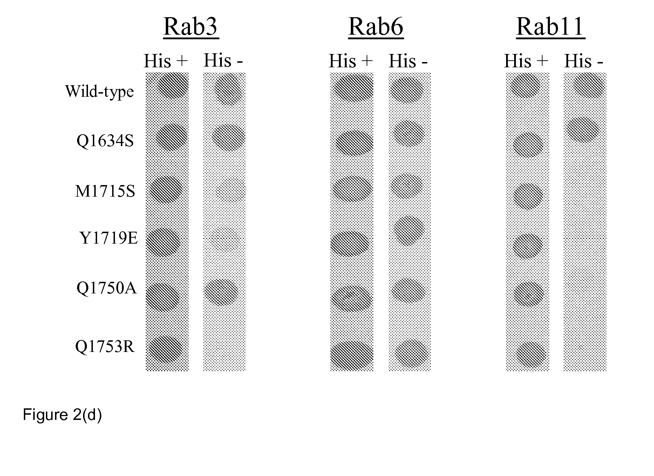

As illustrated in **Figure 2(d)**, the yeast two-hybrid system was used to determine which residues in the myosin Va protein sequence are important for binding Rab GTPases Rab3, Rab6, and Rab11. The control for the experiment is the MyoVa₍₁₁₀₀₋₁₈₅₃₎ domain of wild-type myosin Va. The amino acids essential for myosin Va/Rab GTPase interaction are Q1753 and to a lesser extent M1715 and Y1719 for Rab3, and M1715, Y1719, Q1750, and Q1753 for Rab11. It would appear from the yeast-two hybrid experiment that none of the residues tested were important for the interaction of myosin Va with Rab6 as neither one of the amino acid mutations had any effect on the interaction of Rab 6 and the MyoVa₍₁₁₀₀₋₁₈₅₃₎ domain of wild-type myosin Va.

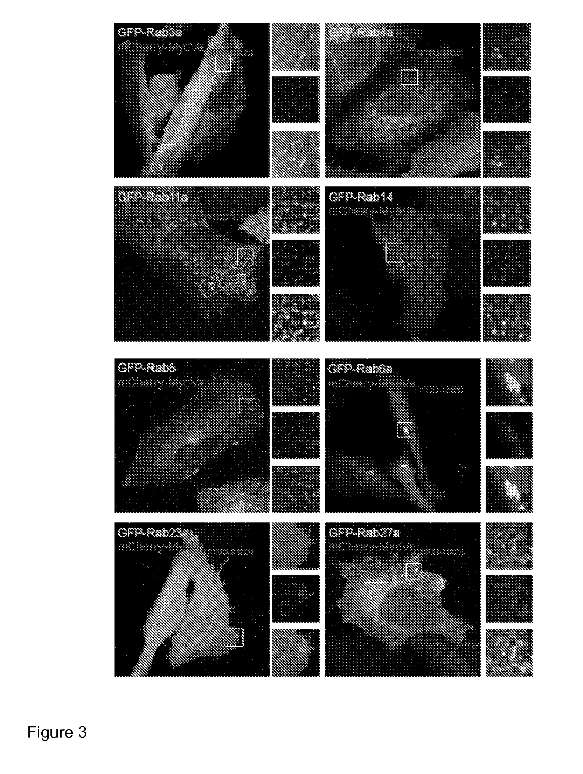

To investigate the subcellular localisation of Rab-GTPases, HeLa cells were transiently transfected with a vector expressing an mCherry-MyoVa₍₁₁₀₀₋₁₈₅₃₎ fusion protein and a vector selected for expression of GFP-tagged Rab3a, Rab4a, Rab5, Rab6a, Rab11a, Rab14, Rab23, and Rab27a proteins. **Figure 3** clearly shows that the mCherry-MyoVa₍₁₁₀₀₋₁₈₅₃₎ fusion protein and GFP-tagged Rabs 3a, 6a, 11 a and 11 b ,14, 23, 25 and 27a colocalise to the same cell compartments. Colocalisation between mCherry-MyoVa₍₁₁₀₀₋₁₈₅₃₎ and GFP-Rab3(WT) is in small vesicles distributed throughout the cell but with concentrations at the cell periphery. These vesicles may be secretory vesicles. MyoVa₍₁₁₀₀₋₁₈₅₃₎ and Rab6a colocalise at or near the Golgi apparatus. There is almost 100% colocalisation between MyoVa₍₁₁₀₀₋₁₈₅₃₎ and Rab11a, Rab11b, and Rab25. vesicles distributed throughout the cell. These vesicles are likely to belong to the endocytic recycling pathway. There is also colocalisation the MyoVa₍₁₁₀₀₋₁₈₅₃₎ and Rab14, and Rab27a. The minor colocalisation with Rab14 is on vesicles, whereas there is a greater amount of colocalisation with Rab27a on secretory vesicles. There is no colocalisation between MyoVa₍₁₁₀₀₋₁₈₅₃₎ and Rab4 or Rab5. These results are significant in that myosin Va and the Rab GTPases identified in the yeast two-hybrid assay to interact with it, also localise to the same structures within the cell. If two proteins interact *in vitro* but are found at different locations within the cell it is unlikely that they will interact *in vivo.* Therefore these colocalisation studies provide further evidence that the interactions are valid.

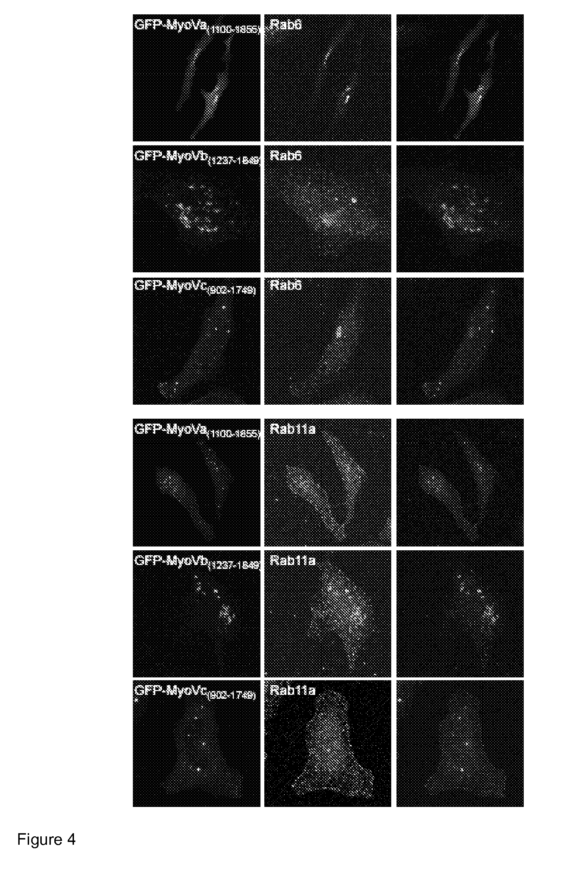

To determine if particular myosin V tails colocalised with endogenous Rab6 and Rab11, HeLa cells were transiently transfected with a vector expressing MyoVa₍₁₁₀₀₋₁₈₅₃₎, MyoVb₍₁₂₃₇₋₁₈₄₉₎, and MyoVc₍₉₀₂₋₁₇₄₉₎ domains fused to GFP. **Figure 4** clearly shows that only cells expressing MyoVa₍₁₁₀₀₋₁₈₅₃₎ colocalised with Rab6, while MyoVa₍₁₁₀₀₋₁₈₅₃₎ and MyoVb₍₁₂₃₇₋₁₈₄₉₎ colocalised with Rab11. No colocalisation was observed with the MyoVb tail for Rab6 and MyoVc tail for both Rab6 and Rab11. This data again provides further evidence that the interactions identified in the yeast two-hybrid screen are genuine. Transfection with the myosin V constructs results in much higher than normal levels of the class V myosin in the cell, this in turn would be expected to recruit the endogenous Rabs to the GFP-fused protein. This is the case for Rab11 a with GFP-MyoVa₍₁₁₀₀₋₁₈₅₃₎ and GFP-MyoVb₍₁₂₃₇₋₁₈₄₉₎ but not with GFP-MyoVc₍₉₀₂₋₁₇₄₂₎, Endogenous Rab6 also colocalises with GFP-MyoVa₍₁₁₀₀₋₁₈₅₃₎ but not with the other Myosin V constructs. This is as expected given the yeast two-hybrid results.

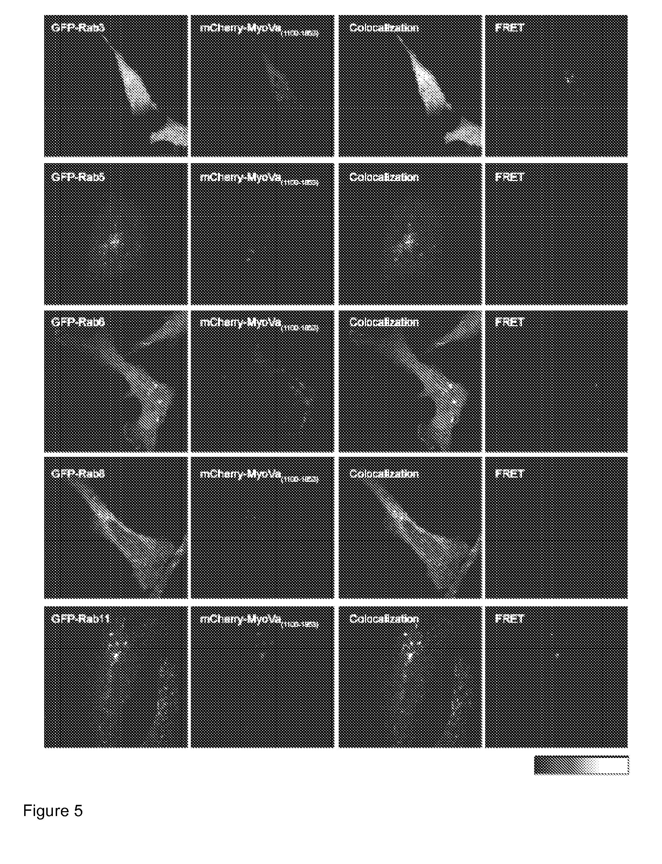

In **Figure 5**, FRET microscopy was used to determine the colocalisation of myosin Va and the Rab GTPases Rab3, Rab5, Rab6, Rab8, and Rab11. FRET microscopy relies on the ability to capture weak and transient fluorescent signals efficiently and rapidly from the interactions of labeled molecules in single living or fixed cells. FRET microscopy has an additional advantage over conventional macroscopic solution measurements of FRET in that the spatial distribution of FRET efficiency can be visualized throughout the image, rather than registering only an average over the entire cell or population. A FRET signal corresponding to a particular location within a microscope image provides an additional magnification surpassing the optical resolution (-0.25 µm) of the light microscope. Thus, within a voxel of microscopic resolution, FRET resolves average donor-acceptor distances beyond the microscopic limit down to the molecular scale. As indicated in **Figure 5****,** HeLa cells were co-transfected with vectors expressing the indicated EGFP-Rab and mCherry-MyoVa₍₁₁₀₀₋₁₈₅₃₎ fusion protein constructs. FRET was determined by the sensitised emission method. It is evident that FRET was only observed for HeLa cells co-expressing the mCherry-MyoVa₍₁₁₀₀₋₁₈₅₃₎ fusion protein and either Rab3, Rab6, or Rab11. No FRET was observed when a HeLa cell co-expressed either Rab5 or Rab8 and the mCherry-MyoVa₍₁₁₀₀₋₁₈₅₃₎ fusion protein. FRET (Förster Resonance Energy Transfer) is a technique that can be used to identify protein-protein interactions within cells. It relies on the ability of certain fluorophore pairs (in this case EGFP and mCherry) with overlapping emission and excitation spectra to transfer energy from one fluorophore to the other. This transfer of energy will take place only when the two fluorophores are within less than 10nm of each other. One of each fluorophore is attached to the proteins of interest and if they interact it will bring the fluorophores into close enough proximity to each other for energy transfer to occur. This is an indication of an interaction occurring between the proteins of interest. The advantage of FRET is that the interaction of two proteins can be examined inside the cell and it will also indicate the location at which the interaction takes place. The results depicted in Figure 5 demonstrate that an interaction between mCherry- MyoVa₍₁₁₀₀₋₁₈₅₃₎ and GFP-Rab3, GFP-Rab6, and GFP-Rab11 takes place on large vesicular aggregates. These aggregates are likely to form due to the overexpression of the fusion proteins but are likely to be derived from the individual compartments at which the individual Rabs reside, i.e. endosomes and Golgi apparatus.

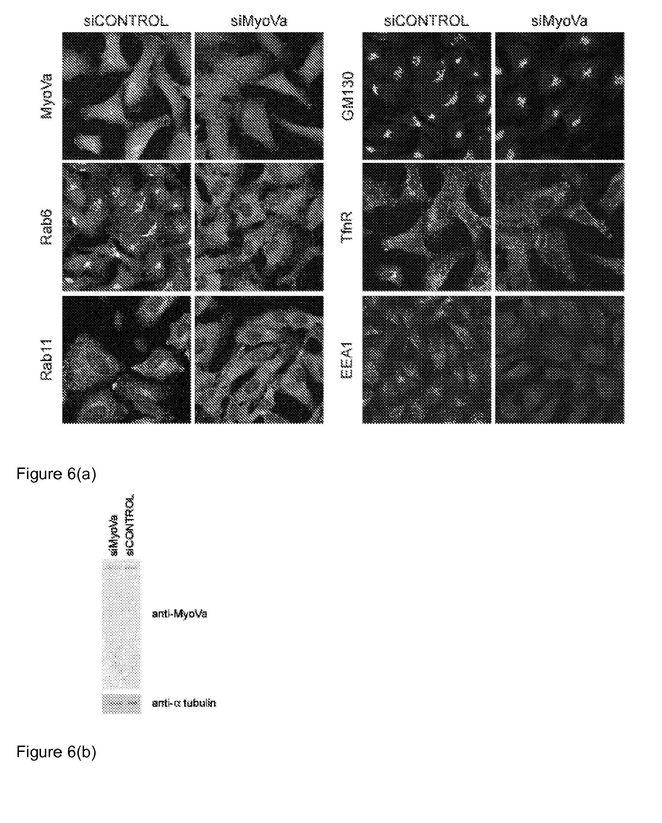

To determine the effect of an absence of myosin Va HeLa cells were transfected with siRNA synthetic oligonucleotides (siMyoVa1 or siMyoVa2) directed against different regions of the myosin Va sequence or a scrambled control for 72 hours prior to fixing and labelling. Quantitative Western blot assays have determined that both siMyoVa1 and siMyoVa2 reduce the level of myosin Va protein by greater than 85%. The siRNA oligonucleotide directed against myosin Va, and used to obtain the results shown in **Figure 6(a)**, is herein referred to as siMyoVa1 and a scrambled control siRNA oligonucleotide sequence is hereinafter referred to as siCONTROL (as per Table 2 above). The siMyoVa1-transfected cells reduced the expression levels of myosin Va by greater than 80% as assessed by Western Blot (**Figure 6(b)**) while those expressing the control siCONTROL had no effect on reducing the expression levels of myosin Va, as shown in **Figure 6(b)**. Myosin Va knock-down had a mild effect on the intracellular localisation of endogenous Rab11 a and transferrin receptor - both proteins displayed slightly less perinuclear localisation. The most dramatic effects of myosin Va knockdown was on EEA1 and Rab6. The EEA1 signal was greatly reduced, and Rab6 labelling of the Golgi was also dramatically reduced. The Golgi itself was unaffected as determined by GM130 (Golgi marker) labelling. Western blots revealed that this was due to the reduced levels of both Rab6 and EEA1 in the myosin Va knockdown cells. (**Figure 6(a)**).

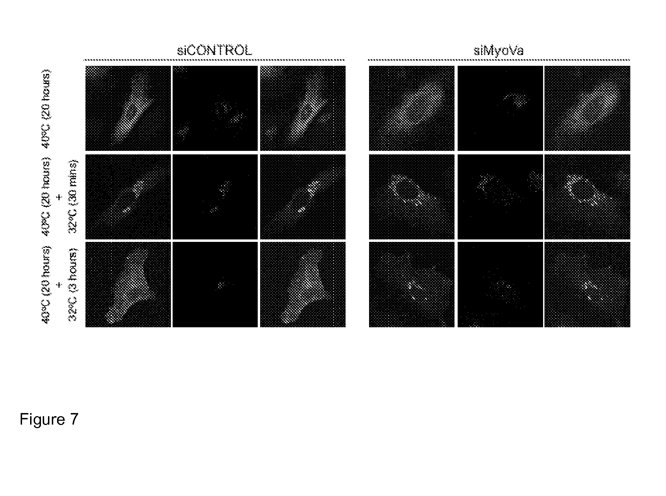

To determine the effect of knockdown expression of myosin Va on vesicle transport from the ER to the plasma membrane, HeLa cells expressing Venus-VSVG were transfected with siMyoVa1 or siCONTROL (**Figure 7**). Cells were placed at 40°C for 20 hours to block Venus-VSVG in the ER. The cells were then either fixed or incubated at 32°C for 30 minutes or 3 hours. When the cells were fixed immediately after the 40°C incubation period, the transportation of VSVG was inhibited between the ER and the plasma membrane. Following incubation at 32°C for 30 minutes or 3 hours, those cells transfected with siCONTROL exhibited transportation of Venus-VSVG from the ER to the Golgi apparatus (30 minutes) and on to the plasma membrane (120-180 minutes). These are the normal timescales for VSVG transport to the cell surface. However, those cells transfected with siMyoVa1 exhibited no inhibition of transportation from the ER to the Golgi, but significant inhibition of onward VSVG transport from the Golgi to the plasma membrane. These results suggest that myosin Va plays a role in the transport step from the Golgi to the plasma membrane.

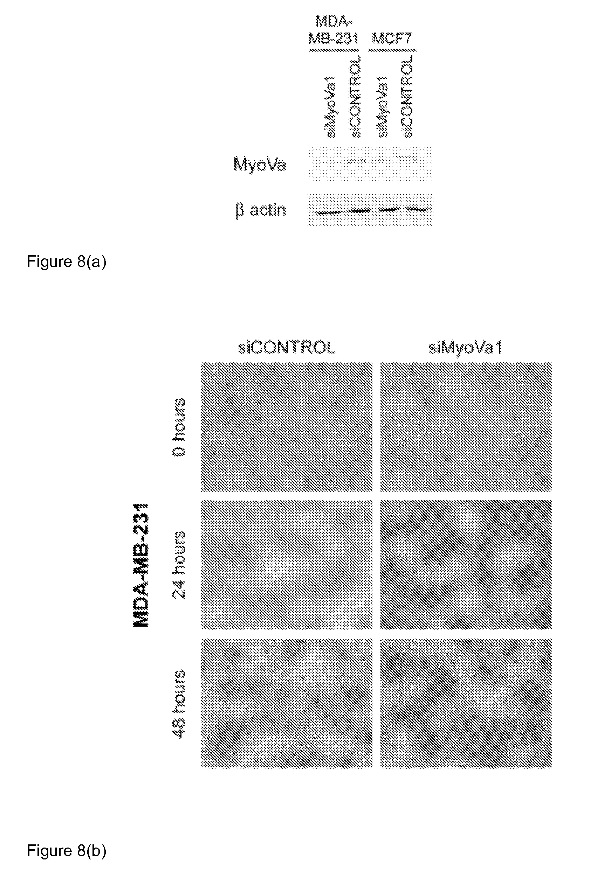

**Figure 8** illustrates the effect inflicting a wound to confluent siCONTROL and siMyoVa1-transfected metastatic breast cancer cell line (MDA-MB-231) in a wound assay. As can be clearly seen, 24 and 48 hours following wound infliction, the cells transfected with siCONTROL exhibited no inhibition in cell migration. However, those cells transfected with siMyoVa1 exhibited a reduced rate of migration when compared to control. Therefore, myosin Va plays an important role in the metastatic nature of, in this instance, breast cancer cells. Therefore, a number of classes of compounds may be used to treat or prevent a variety of diseases and/or conditions associated with malfunctioning or compromised vesicle transport systems in cells. Such compounds may comprise:
(i) small cell-penetrating peptides homologous to the Rab3, Rab11, and Rab6 interacting domains of myosin Va which may be used to selectively disrupt individual myosin Va-Rab interactions, the malfunctioning of which are associated with diseases having cargo transport deficiencies in the cell. Such peptides will compete with myosin Va by binding to the individual Rab GTPases;
(ii) small-molecule inhibitors that selectively block the Rab-binding pockets of myosin Va, thus preventing interaction with individual Rab GTPases
(iii) siRNA oligonucleotides to modulate the level of myosin Va expression. Both myosin Va siRNA oligonucleotides listed herein reduce the levels of myosin Va protein by greater than 80% as assessed by Western blot and immunofluorescence of transfected human cervical and breast cancer cell lines; and
(iv) Monoclonal antibodies that bind to and inhibit the functioning of myosin Va.

One skilled in the art would readily understand that the methods for detecting binding of a myosin Va Rab binding domain (myosin Va RBD) and a compound such as those listed above can be any method known in the art. An interaction between a myosin Va RBD and a compound listed above, for example, could be detected using:

### Screening In Vitro

Dual Polarisation Interferometry (DPI) - myosin Va RBD expressed as a His- or GST-fusion protein bound to an appropriate chip and screened for binding of potential compounds by dual polarisation interferometry (DPI) using the Farfield Analight system available in the lab. This system has the ability to detect the binding of molecules of less than 50Da to immobilised proteins. Alternatively screening may be performed by surface plasmon resonance (SPR) using a Biacore system in collaboration with a suitable laboratory.

### Screening In Vivo

Generation of a FRET 'biosensor'. A donor fluorophore (e.g. CFP or EGFP) will be fused to the amino-terminal domain of the myosin Va Rab-binding domain (RBD) and an acceptor fluorophore (e.g. YFP or mcherry) will be placed at the carboxy-terminus. Binding of a compound to the myosin Va RBD would be expected to alter the conformation of the protein and thus result in a measurable alteration of the fluorescence energy transfer from the donor fluorophore to the acceptor fluorophore.

Biotinylated peptide. In the case of a peptide inhibitor, the peptide could be biotinylated and delivered into the cell for a defined period of time. The cells would then be lysed and the peptide retrieved with streptavidin-conjugated magnetic beads. Bound myosin Va RBD would be detected by Western blotting.

HRP-conjugated compound. The compound may be conjugated to horseradish peroxidase (HRP) and delivered into the cell by standard means. Its location within the cell with respect to myosin Va could be determined by immunoelectron microscopy.

Identical experiments to those carried in Figures 6-8 using siMyoVa1 were also performed using siMyoVa2. The results obtained for siMyoVa2 were identical to those results obtained for siMyoVa1 but are not shown here.

### Discussion

The results observed to date demonstrate that myosin Va interacts directly with Rab3, Rab6, and Rab11. siRNA-mediated knock-down of myosin Va in conjunction with cargo transport assays indicate that myosin Va functions in transport steps from the Golgi apparatus to the plasma membrane. This correlates well with the observed interactions with the Rab GTPases as each of the interacting Rabs have been independently shown to be involved in these steps also. Rab6 - from Golgi apparatus to the plasma membrane either directly, or indirectly via the endocytic recycling compartment (ERC). Rab11 - from the ERC to the plasma membrane or from the ERC to the Golgi. Rab3 - Rab3 is expressed in a subset of cell types and has been implicated in the fusion of transport vesicles with the plasma membrane. In summary it would appear that myosin Va is involved in the transport of cargo to the plasma membrane from the Golgi and/or the ERC to the plasma membrane. This pathway is extremely important for the delivery of a plethora of cargos to the cell surface including GPCRs, receptor tyrosine kinases, AMPA receptors, integrins, glucose transporters (GLUTs) etc. This pathway is also 'hijacked' by a number of viruses during their cell cycle. We postulate that the delay in wound closure observed upon myosin Va knock-down in MDA-MB-231 cells is due to an inhibition in the transport of certain integrins to the cell surface.

To determine if myosin Va can associate with more than just Rab27a the yeast two-hybrid system was used to test the carboxy-terminal tail of human myosin Va for interactions with a range of Rab GTPases. Direct interactions with the GTP-bound forms of several Rab GTPases of the exocytic pathway were observed. These interactions could be mapped to a cluster of amino acids in the globular tail domain of myosin Va, and were not mediated by the alternatively-spliced exons B or F. Myosin Va was found to colocalise with these small GTPases in various cell lines and fluorescence resonance energy transfer (FRET) was observed between mCherry-tagged myosin Va tail and the green fluorescent protein (GFP)-tagged Rab GTPases. It is emerging that the class V myosins are essential for the final steps in the transport of cargo such as integrins, G-protein coupled receptors (GPCRs), receptor tyrosine kinases (RTKs), etc, to the cell surface. The inventors of the present invention found that myosin Va interacts directly with Rabs 3, 6, and 11. These three small GTPases have each been implicated in regulating cargo transport along the secretory pathway. RNAi-mediated knockdown of myosin Va perturbs the localisation of Rab6 and inhibits the exit of cargo from the Golgi. Taken together this data suggests that myosin Va is involved in the delivery of cargo to the plasma membrane and that its role in the secretory pathway is regulated by these Rab GTPases.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A method for screening compounds to identify therapeutic candidates for the treatment of a disease associated with myosin Va malfunction, said method comprising:
(i) contacting a compound with a cell expressing a myosin Va Rab binding domain under conditions which permit binding of a myosin Va Rab binding domain to a Rab protein; and
(ii) detecting binding between a myosin Va Rab binding domain and a compound;
wherein binding between a myosin Va Rab binding domain and a compound indicates that a compound is a candidate for modulating the interaction between myosin Va and a Rab protein, and regulating the function of myosin Va in the transport of cellular proteins to a cell surface *in vivo.*

2. A method according to Claim 2, wherein the Rab-binding domain of myosin Va is encoded by a polynucleotide and/or a polypeptide selected from the group comprising:
(a) a polynucleotide encoded by SEQ ID NO:1;
(b) a polynucleotide encoding the amino acid sequence as defined by SEQ ID NO:2;
(c) a polynucleotide complementary to a polynucleotide of (a) or (b);
(d) a polypeptide encoded by SEQ ID NO: 2;
(e) amino acid residues 1100 to 1853 of SEQ ID NO: 2;
(f) amino acid residues 1100 to 1853 of SEQ ID NO: 2 having one or several amino acid additions, substitutions, insertions or deletions; and
(g) an amino acid sequence which hybridises under stringent conditions to any one of (d), (e), or (f);
wherein each of (f) to (g) have Rab protein binding activity.

3. A method according to Claim 1 or Claim 2, wherein the interaction between the Rab protein and myosin Va is dependent on amino acid residues L1633, M1715, Y1719, Q1750, and Q1753 of SEQ ID NO: 2 or a polynucleotide encoding said amino acid residues.

4. A method according to any of Claim 1 to Claim 3, wherein the Rab protein is selected from the group comprising Rab3a, Rab6a, Rab6a', Rab11a, Rab11b, and Rab25.

5. A method according to any of Claim 1 to Claim 4, wherein the compound is selected from the class of compounds comprising (i) small cell-penetrating peptides homologous to the Rab3, Rab11, and Rab6 interacting domains of myosin Va; (ii) small-molecule inhibitors that selectively block the Rab-binding pockets of myosin Va; (iii) siRNA oligonucleotides to modulate the level of myosin Va expression; and (iv) monoclonal antibodies that bind to and inhibit the functioning of myosin Va.

6. A polynucleotide sequence encoding a myosin Va Rab binding domain selected from the group comprising:
(a) amino acid residues 1100 to 1853 of SEQ ID NO: 2;
(b) amino acid residues 1100 to 1853 of SEQ ID NO: 2 having one or several amino acid additions, substitutions, insertions, or deletions; and
(c) an amino acid sequence which hybridises under stringent conditions to any of (a) or (b);
wherein each of (b) and (c) have Rab protein-binding activity.

7. A polynucleotide sequence encoding a myosin Va Rab binding domain comprising amino acid residues L1633, M1715, Y1719, Q1750, and Q1753 of SEQ ID NO: 2 or a polynucleotide encoding said amino acid residues.

8. A pharmaceutical composition comprising a peptide or nucleic acid encoding a myosin Va Rab-binding domain.

9. The composition according to Claim 8 wherein the Rab-binding domain of myosin Va is a polynucleotide and/or a polypeptide selected from the group comprising:
(a) a polynucleotide encoded by SEQ ID NO:1;
(b) a polynucleotide encoding the amino acid sequence as defined by SEQ ID NO:2;
(c) a polynucleotide complementary to a polynucleotide of (a) or (b);
(d) a polypeptide encoded by SEQ ID NO: 2;
(e) a polynucleotide sequence according to Claim 6; and
(f) a polynucleotide according to Claim 7.

10. A composition according to Claim 8 or Claim 9, wherein the interaction between a Rab protein and myosin Va is dependent on amino acid residues L1633, M1715, Y1719, Q1750, and Q1753 of SEQ ID NO: 2 or a polynucleotide encoding said amino acid residues.

11. A composition according to any of Claim 7 to Claim 9, wherein the Rab protein is selected from the group comprising Rab3a, Rab6a, Rab6a', Rab11 a, Rab11 b, and Rab25.

12. A composition according to any of Claims 8 to 11 further comprising a pharmaceutically effective amount of an agent which modulates the interaction between myosin Va and a Rab protein.

13. A composition according to Claim 12, wherein said agent is selected from the group comprising (i) small cell-penetrating peptides homologous to the Rab3, Rab11, and Rab6 interacting domains of myosin Va; (ii) small-molecule inhibitors that selectively block the Rab-binding pockets of myosin Va; (iii) siRNA oligonucleotides to modulate the level of myosin Va expression; and (iv) monoclonal antibodies that bind to and inhibit the functioning of myosin Va.

14. Use of an effective amount of a compound identified by the method of any of claims 1 to 5 in the manufacture of a medicament to treat or prevent a disease.

15. A vector for use in treating or preventing a disease by gene therapy, the vector comprising a polynucleotide and/or polypeptide sequence selected from any of Claim 2, Claim 6, or Claim 7.
